(19) Europäisches Patentamt
European Patent Office
Office européen des brevets

(11) **EP 2 163 271 B1**

(12) **EUROPEAN PATENT SPECIFICATION**

(45) Date of publication and mention
of the grant of the patent:
**11.04.2012 Bulletin 2012/15**

(51) Int Cl.:
***A61M 1/16*** (2006.01)

(21) Application number: **08016170.6**

(22) Date of filing: **15.09.2008**

(54) **Method to determine the Kt/V parameter in kidney substitution treatments based on a non-linear fitting procedure**

Verfahren zur Bestimmung des Kt/V-Parameters bei Nierenersatzbehandlungen basierend auf einem nicht-linearen Passungsverfahren

Procédé pour déterminer le paramètre Kt/V dans des traitements de remplacement de rein basé sur une procédure d'adaptation non linéaire

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**17.03.2010 Bulletin 2010/11**

(73) Proprietor: **B. Braun Avitum AG**
**34212 Melsungen (DE)**

(72) Inventors:
• **Castellarnau, Alex**
**34212 Melsungen (DE)**
• **Ahrens, Jörn**
**34225 Baunatal (DE)**
• **Moll, Stefan**
**34212 Melsungen (DE)**
• **Wagner, Jürgen**
**34131 Kassel (DE)**

(74) Representative: **Arth, Hans-Lothar**
**ABK Patent Attorneys**
**Jasminweg 9**
**14052 Berlin (DE)**

(56) References cited:
**EP-A1- 1 698 360**      **WO-A1-97/44072**
**WO-A1-98/23311**      **WO-A1-98/55166**

• **CANAUD B; ET AL: "ON-LINE DIALYSIS QUANTIFICATION IN ACTUTELY III PATIENTS. PRELIMINARY CLINICAL EXPERIENCE WITH A MULTIPURPOSE UREA SENSOR MONITORING DEVICE" ASAIO JOURNAL, LIPPINCOTT WILLIAMS & WILKINS / ASAIO, HAGERSTOWN, MD, US, vol. 44, no. 3, 1 May 1998 (1998-05-01), pages 184-190, XP000752086 ISSN: 1058-2916**

**Description**

The invention relates to a method to determine the Kt/V parameter in kidney substitution treatment.

Dialysis adequacy is the topic that has got and gets more attention when one thinks about patient outcome. In order to estimate dialysis adequacy one needs a parameter establishing a relation between dialysis dosage and patient outcome. The most accepted parameter to estimate the quantity of dialysis delivered or dosage is the Kt/V, where K is the effective clearance for urea, t is the treatment time and V is the urea distribution volume which matches the total body water.

The NCDS (National Cooperative Dialysis Study) and the HEMO study found, after analyzing a large patient group, that morbidity and mortality in end stage renal disease (ESRD) was strongly correlated with the Kt/V value or dialysis dose. Data obtained from these studies resulted in guidelines regarding hemodialysis treatments, which demand a minimum dose of Kt/V=1.2 generally and 1.4 for diabetics respectively (DOQI guidelines).

It is worthy to point out that a morbidity decrease not only improves the patient well-being, but also reduces significantly the medical costs as the patient requires less care.

The need of a reliable and cost effective method to monitor the Kt/V and by extension control dialysis adequacy and morbidity, is therefore easily understood.

In the Kt/V calculation, the main problems are K and V estimation along with the multi-compartment urea kinetics. V can be estimated by bioimpedance, anthropometric measurements or applying the urea kinetic model (UKM), all these methods have a certain degree of error. K can be estimated so far by measuring the urea blood concentration before and after the treatment or by monitoring inlet and outlet conductivity changes in the dialysate side.

Blood samples method is the reference one. After taking the blood samples and applying either UKM or Daugirdas formula a single pool Kt/V (spKt/V) is estimated, further, Daugirdas second generation formulas should be used to get an equilibrated Kt/V (eKt/V) which accounts for the urea rebound caused by the fact that urea kinetic's does not follow a single pool model but a multi-compartment one. This method has two main problems: it is not possible to know whether the treatment is adequate or not before it finishes, therefore it is not possible to perform any action to improve the situation; it is not an easy to apply method: sampling time is very important to get an accurate value, and the medical staff must send the samples to the lab, wait for the results and calculate Kt/V values with the help of a computer. These facts result on a monthly basis Kt/V measurements in best case, which means that in worst case scenario a patient might be under-dialyzed for one whole month.

Conductivity methods are based on the observation that sodium clearance is almost equal to urea clearance and that the relationship between dialysate conductivity and dialysate sodium concentration can be considered linear on the temperature range of interest. Therefore it is possible to get urea clearance by measuring the sodium diffusion transport through the membrane in the dialyzer.

It is important to introduce the concept of Dialysance, as it slightly differs from Clearance:

Clearance is defined as the ratio between transport rate and concentration multiplied by flow, and it is applicable when the diffusing substance is on the blood side but not on the dialysate, that is the case for urea.

Dialysance is defined as the ratio between transport rate and concentration gradient multiplied by flow, and it is applicable when the diffusing substance is in both dialyzer sides. When one applies conductivity methods to measure urea Clearance, one actually measures sodium Dialysance (Depner T, Garred L. Solute transport mechanisms in dialysis. Hörl W, Koch K, Lindsay R, Ronco C, Winchester JF, editors. Replacement of renal function by dialysis, 5th ed. Kluwer academic publishers, 2004:73-91).

During conductivity based clearance measurements, a dialysate inlet conductivity different to the blood one is produced, which results in a net transfer of sodium either from blood to dialysate or from dialysate to blood due to the generated gradient. There are currently several methods which are applied in the industry:

In a first method in a first step a conductivity profile is determined and in a second step the conductivity profile is used for integration of conductivity peaks (Polaschegg HD, Levin NW. Hemodialysis machines and monitoris. Hörl W, Koch K, Lindsay R, Ronco C, Winchester JF, editors. Replacement of renal function by dialysis, 5th ed. Kluwer academic publishers, 2004:414-418). The main advantages of such approaches are: they are relatively easy to implement and cost effective as they only need an extra conductivity/temperature sensor downstream the dialyzer; they offer Kt/V measurements during the treatment allowing the medical staff to react and perform some actions in case the treatment is not going as it should.

However, conductivity based methods have also some limitations: they can induce some sodium load in the patient during the measurement; they are not useful to obtain other interesting parameters like nPCR or TRU; the maximum measurement frequency offered so far by the industry is about 20 minutes, that means that in worst case scenario the patient could be under-dialyzed for 20 minutes; and, although there are some publications and patents regarding it, so far, conductivity methods haven't been applied with enough reliability to hemofiltration or hemodiafiltration treatments.

Another method to estimate hemodialysis adequacy is by direct measurement of the waste products (urea) concentration in the effluent dialysate, this method assumes that the evolution of urea concentration over the time in the dialysate side is proportional to the one in the blood, therefore the slope of the line obtained after applying the natural logarithm to the registered concentration values over the time will be the same on both sides: dialysate and blood, and by definition such slope is K/V, which multiplied by the therapy time results on the Kt/V value.

There are two methods available to measure online the concentration of waste products in effluent dialysate: Urea sensors and UV spectrophotometry.

The limitations of the urea sensors are well known. Recent works carried out by Fridolin I. et al (I. Fridolin, M. Magnusson, L.-G. Lindberg. On-line monitoring of solutes in dialysate using absorption of ultraviolet radiation: Technique description. The International Journal of Artificial Organs. Vol. 25, no. 8, 2002, pp. 748-761) and Uhlin F. (Uhlin F. Haemodialysis treatment monitored online by ultra violet absorbance. Linköping University Medical Dissertations n° 962. Department of Medicine and Care Division of Nursing Science & Department of Biomedical Engineering. 2006.) have shown that UV spectrophotometry is a reliable and cost affordable method to monitor waste products in effluent dialysate. Additionally the European Patent EP1083948B1 describes a sensor coupled with the dialysate flow system of a dialysis machine, which is actually an UV spectrophotometer measuring UV absorbance of UV absorbing products in spent dialysate.

The author has found that the above referenced works have however some limitations:

- The logarithmic linearization of the timely exponential decay of the concentration of waste products on spent dialysate leads to a linear least squares fitting procedure. Recent experiences showed that the Kt/V values obtained by said procedure are about 10% lower than the Kt/V values obtained by means of blood samples.

- The clearance and consequently the Kt/V value during a dialysis treatment strongly depend on the counter current effect between patient blood and dialysis fluid, thus changes on the blood and/or dialysate flow will affect the treatment clearance and Kt/V. Said flow changes also affect the timely exponential decay of waste products on spent dialysate, however due to the inherent noise of the measurement and the relative low gain that the exponential decay has, it is almost impossible to recognize these flow changes by analyzing the decaying concentration curve, specially if the changes are not dramatic.

- From the kinetics point of view is it possible to say that the human body has two compartments, intracellular (IC) and extracellular (EC). At the beginning of any kidney substitution treatment, the machine clears the EC, creating a gradient effect between IC and EC, which forces the waste products from the IC diffuse and/or convect into the EC. General speaking the clearing capabilities of any dialyzer are higher than the existing clearance between IC and EC, therefore the second will limit the clearance of the first. Such situation results in the fact that the timely decrease of the blood urea concentration during a dialysis treatment does not follow a simple exponential decay, but a double exponential decay, wherein one of the exponential factors depends on the clearance between IC and EC, and the other depends on the dialyzer clearance.

The inherent assumption of a single exponential decay of the above referenced works leads to noticeable inaccuracies when one compares the result of the measuring with the Kt/V obtained by means of blood samples.

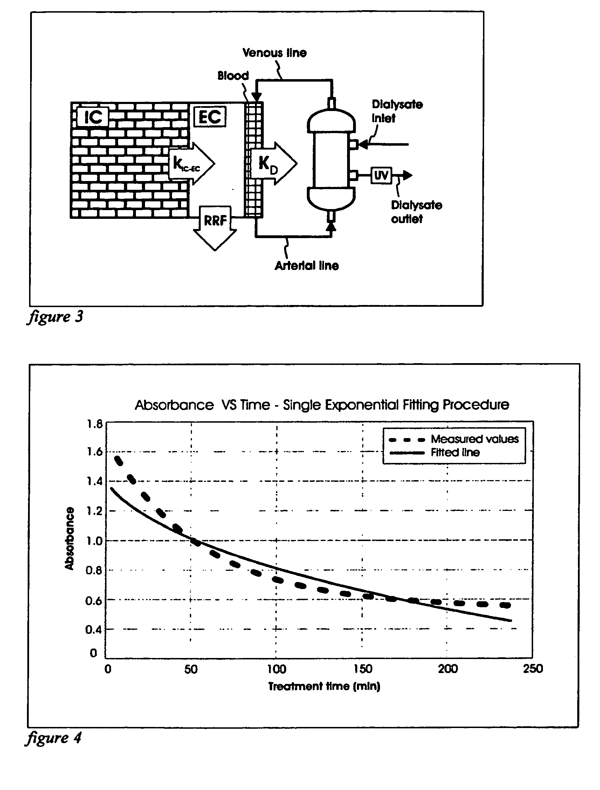

FIG. 3 depicts the body compartments which are important from the kinetics point of view and the relations among them. The clearance $k_{IC-EC}$ between IC and EC controls the diffusion and convection of waste products from IC into EC. As long as the solutes are in the EC, which comprises interstitial fluid and blood, they can be cleared from the body either by the residual renal function (RRF) or the dialysis treatment. The clearance due to the treatment is represented on the draw as $K_D$. The UV sensor is coupled with the dialysate outlet.

An example of prior art method is given in WO-98/55166.

The method of the present invention differs from said prior art in that measurement data from a data measurement device is splitted in subsets, and a data interface is provided between the kidney substitution treatment machine and the measurement device. The aim of this invention is to provide a reliable method, which in combination with any device able to continuously measure any dialysis related waste product, overcomes the above listed problems. Said measuring device may be coupled at any position of the flow system of a dialysis machine.

This problem is solved by a method with the features described in claim 1. Preferred embodiments of the invention are described in the claims 2 to 16.

Further goals, advantages, features and possibilities of use of this invention arise out of the subsequent description of the embodiments of the invention. Therefore every described or depict feature of its own or in arbitrary meaningful combination forms the subject matter of the invention even independent of its summary in the claims or its reference to other claims.

It shows:

FIG 1 Depicts a portion of a conventional dialysis machine plus a slight modification to host a sensor coupled with the dialysate circuit,

FIG 2 Depicts a standalone measuring device coupled with a dialysis machine,

FIG 3 Depicts the body compartments regarding urea kinetics and the relationships among them,

FIG 4 Graph depicting the absorbance measured during a real treatment (dotted) together with a fitted line (solid) modelled using a single exponential function,

FIG 5 Graph depicting the absorbance measured during a real treatment (dotted) together - with a fitted -line (solid)-modelled using a double exponential function and

FIG 6 Graph depicting the absorbance measured during a real treatment (dotted) together with a fitted line (solid) modelled by splitting the whole dataset in 12 subsets and applying a single exponential fitting procedure to each of them.

**Description of preferred embodiments**

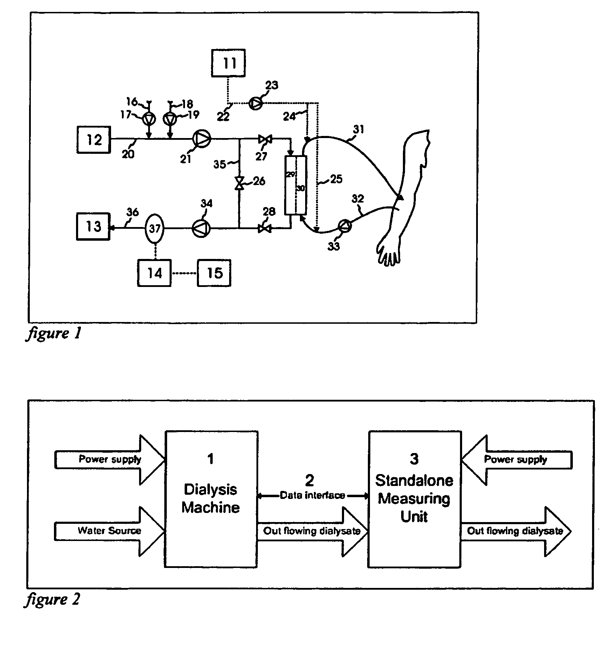

FIG. 1 shows a draw of the dialysate circuit of a conventional dialysis machine plus a slight modification to host a sensor coupled with the dialysate circuit.

The blood from a patient is taken out into an extracorporeal circuit, it flows through the tube 32 into the blood chamber 30 of a dialyzer and returns to the patient through the tube 31. The flow rate of the blood circuit is controlled by the blood pump 33.

The dialysis fluid is made of several concentrates and water, therefore the machine disclosed in figure 1 comprises a water inlet 12, two concentrates inlets 16 and 18 and two concentrate pumps 17 and 19. The water flow together with the concentrates flow define the final properties of the dialysis fluid.

The conduit 20 takes the dialysis fluid to the dialysate chamber 29 of the dialyzer, which is separated from the blood chamber 30 by a semipermeable membrane. The dialysis fluid it is pumped into the dialyzer by the pump 21. A second pump 34 sucks the dialysis fluid and any ultrafiltrate removed from the blood.

A bypass line 35 is arranged between the pumps 21 and 34. Several valves 26, 27 and 28 are arranged to control the dialysate flow.

The conduit 36 leads the spent dialysate to a UV-sensor 37 measuring its light absorbance, the UV-sensor 37 is connected by an interface with the computer 14 which processes the measured data, the result of the data processing is displayed and/or printed by the device 15, which is connected with the computer 14 by an interface.

The conduit 36 leads the spent dialysate after its measurement by the sensor 37 to the drain system 13.

The dotted lines 22, 24 and 25 represent an adaptation of the disclosed apparatus for hemodiafiltration treatments. The substitution fluid comes from a substitution fluid source 11, flows through the line 22 and is pumped in the blood lines of the patient by the pump 23. In case of post dilution hemodiafiltration the conduit 24 leads the substitution fluid to the venous line of the extracorporeal blood system; in case of pre dilution hemodiafiltration the conduit 25 leads the substitution fluid to the arterial line of the extracorporeal blood system; and in case of pre-post dilution hemodiafiltration both conduits 24 and 25 are used.

The computer 14 controls all the elements shown on the figure by means of proper interfaces, said interfaces are not drawn for the sake of simplicity.

The computer 14 gathers information about other parameters of the dialysis machine, like for example blood flow, dialysate flow and/or therapy time, these parameters together with the measured data are processed, the result tunes the Kt/V measuring functionality to assess deviations.

Eventually the UV-sensor 16 can be substituted by an Urea-sensor, in this case will the urea concentration in spent dialysate measured instead of the light absorbance.

The disclosed dialysis machine is provided with several other means as is conventional. These other means are not disclosed, since they are not relevant for the operation of the present invention.

FIG. 2 shows another embodiment where the measuring unit is build in a stand alone device 3 coupled with the effluent dialysate of a dialysis machine 1. An interface 3 between the standalone device and the dialysis machine allows a bidirectional data communication required to minimize the deviation of the Kt/V measuring function.

1. Direct exponential non linear fitting procedure

As described above, the current algorithms linearize the exponential decay of waste products concentration in spent dialysate, apply a linear regression, and work out the Kt/V value. However it is possible to apply a non linear regression procedure to directly model the measured data avoiding the linearization step, such approach significantly improves the measurement accuracy.

The practical application of said non linear fitting procedure comprises a measuring unit coupled with the flow system of a dialysis machine and a software implementation of the non linear regression procedure. The software algorithm keeps a collection of measurements delivered by the measuring unit, when the data collection has at least two values, the algorithm performs successive iterations to minimize the difference between the experimental or measured data and the fitted function. Every time a new value arrives to the data collection the procedure is repeated increasing the accuracy of the model. FIG. 4 shows a graph with successive measurements together with the modelled function which best fits the experimental data.

The modelled function in the preferred embodiment is a single exponential decay of the form:

$$A_t = A_o \cdot e^{\frac{K}{V}t} \quad (1)$$

Where:

- $A_t$ is the UV-Absorbance at treatment time t.
- $A_o$ is the UV-Absorbance at the beginning of the treatment, and the offset of exponential function.
- KN matches the ratio between clearance and urea distribution volume and also the slope of the exponential function. When multiplied by the treatment time results on the Kt/V value at the given treatment time.

A collection of data pairs absorbance-time are the input for the fitting procedure. The outputs are $A_o$ and K/V. The latter multiplied by the treatment time gives the Kt/V value.

It is possible to apply the above described method to any model handing a factor, which meets the Kt/V value or any other dialysis adequacy parameter either directly or by means of mathematical operations, which require as input said modelled factor.

It is also possible to split the data in subcollections, fit each of them individually and apply a mathematical operation with all the partial results to work out the final parameter. In the above described embodiment, where an UV-Spectrophotometer is coupled with the effluent dialysate of a dialysis machine, the whole treatment data may be divided in 4 subcollections, the fitting procedure hands a K/V factor for each of the four collections, said factors multiplied by the time that each subcollection lasts result on four partial Kt/V values, which after addition deliver the Kt/V value for the whole treatment.

The number of subcollections considered by the algorithm depends on empiric data, the purpose is to maximize the accuracy of the measuring system.

2. Single exponential fitting on split measured data

Due to the double compartment nature of the human body the timely decay of waste products concentration in blood and/or spent dialysate do not actually follow a single exponential function but a double exponential function of the form.

$$C_t = a \cdot e^{-bt} + c \cdot e^{-dt} \quad (2)$$

Where one of the factors describes the diffusion and/or convection of waste products between ICM and ECM, and the other between ECM and dialysis machine.

This fact reveals that the method published by Uhlin et al has an inherent error, and could also explain the deviations reported by the same author.

FIG. 4 shows a plot of a timely decrease of waste products absorbance (concentration) in spent dialysate together with a best fitted line using a single exponential model. In this case it is possible to see that, while during most of the treatment the fitted line (solid) is slightly above or follows the actual line (dotted), at the beginning it is clearly below and at the end is again slightly below.

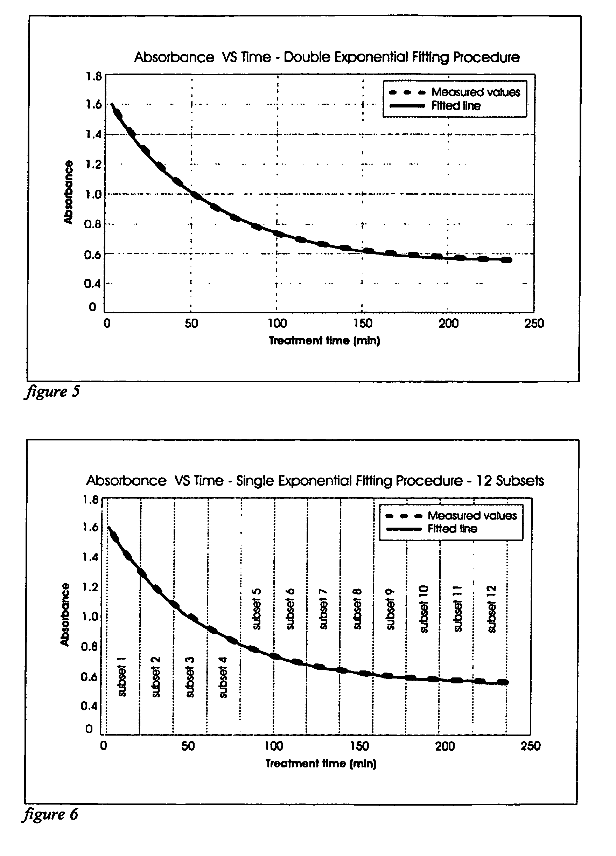

FIG. 5 shows the same data but fitted with a double exponential model, in this case the fitted line (dotted) perfectly follows the actual absorbance line (solid).

Because of mathematical reasons it is not possible to fuse the factors b and d of the equation 2 in one common factor that could eventually match the ratio K/V like in the single exponential model. However it is possible to split the measured or actual line in many pieces, fit a single exponential model and add the resulting partial Kt/Vs into the Kt/V value for the whole treatment in a subcollections like fashion (see above).

FIG. 6 shows again the same data set, after splitting it in twelve pieces and fitting individually each of the pieces using a single exponential model. In that case even using an imperfect model the fitted line (solid) perfectly follows the actual absorbance line (dotted).

Alternatively to the exponential non linear fitting procedure, it is also possible to fit a line using a linear regression with each data subset. The slope of said fitted line matches the K/V ratio as well, which multiplied by the time range of the analyzed data subset results on a partial Kt/V. The whole Kt/V is calculated as described above, by adding up all the obtained partial Kt/Vs. The slight accuracy loss can be compensated by the fact that this procedure is much easier to compute than the previous one.

The number of pieces into which the data is split depends on empiric data, the author has found that a number of pieces between eight and twelve maximize the accuracy of the method.

The described method together with a measuring unit, able to measure the concentration and/or absorbance of any waste product in dialysate, coupled with the flow system of a kidney substitution treatment machine is able to measure the Kt/V value or other adequacy parameters with high accuracy and reproducibility.

3. Data interface between measuring unit and machine

As commented above, the clearance and consequently the Kt/V value during a dialysis treatment strongly depend on the counter current effect between patient blood and dialysis fluid, thus changes on the blood and/or dialysate flow will affect the treatment clearance and by extension the Kt/V and/or other adequacy parameters.

A measuring unit, coupled with the flow system of a dialysis machine and able to continuously measure concentration of waste products in spent dialysate, will reflect in its output, changes on the slope of the timely exponential decay every time that any parameter able to affect the treatment clearance changes, like for example changes on blood flow and/or dialysate flow. Due to the inherent noise of the measurement and the relative low slope that the exponential decay has, it is almost impossible to recognize these flow changes by analyzing the decaying concentration curve, especially if the changes are not dramatic.

However if there is a data interface between the measuring unit and the dialysis machine, because either the measuring unit is integrated on the dialysis machine or, if the measuring unit is a stand alone device, there is a external data interface connecting it with the dialysis machine, any change on any parameter able to affect the clearance can be tracked and the Kt/V function algorithm may use a subcollection like fashion as explained above to split the treatment data in pieces having constant parameters, fit each piece individually, work out partial Kt/V values and add them up to get the Kt/V value or other adequacy parameters for the whole treatment.

In one of the preferred embodiments the UV sensor measures every 3 minutes, of course the measurement frequency could be higher, but it has been found that a measurement frequency of 3 minutes delivers enough data, about 80 measurements in a 4 hours treatment, to measure the Kt/V with enough reliability and accuracy.

If the treatment conditions are kept constant, meaning that there are no blood and/or dialysate flow changes during the dialysis, each of the above mentioned twelve data subsets will include about seven measurements. Therefore twelve partial Kt/Vs will be calculated for each of the subsets and the final whole Kt/V will be the result of adding the partial Kt/Vs up.

If the dialysis parameters that may affect the clearance are changed, like for example blood and/or dialysate flow, the timestamp of the change will be tracked and the current subset will be close even if it does not contain yet seven measurements. The machine will wait for a dead time until to start a new subset.

The dead time is required because of two reasons: The dialysis procedure is a slow system with an important inertia, the new conditions require some time to be stable; the UV sensor is coupled with the out flowing dialysate, therefore the effect of the changes needs some time to flow from the filter to the position of the UV sensor. The latter results on a shifted absorbance curve at the sensor position when comparing it with an hypothetic absorbance curve directly measured at dialyzer outlet. In the following table the first absorbance measurement happened at time 5.1 minutes, however the time considered in the first data subset it is not from 5.1 to 23.3 but 0 to 23.3. Even though there is no data available, during the first 5.1 minutes of treatment the patient is of course treated, so the results are better if the first K/V factor is extrapolated to the whole treatment time, starting at time 0. Similar situation happens when a change on the dialysis condition, a bypass or a sequential period force the start of a new data subset.

The algorithm may work as shown in the following example:

| Measurement n° | Timestamp (min) | UV absorbance | K/V factor | Kt/V |
|---|---|---|---|---|
| 1 | 5.1 | 1.40 | - | - |
| 2 | 8.2 | 1.35 | 0.012 | 0.10 |
| 3 | 11.2 | 1.31 | 0.011 | 0.12 |
| 4 | 15.2 | 1.27 | 0.011 | 0.17 |
| 5 | 17.0 | 1.24 | 0.010 | 0.17 |
| 6 | 20.3 | 1.21 | 0.010 | 0.20 |
| 7 | 23.3 | 1.18 | 0.009 | 0.21 |
| Default closing of first subset (7 measurements). Partial Kt/V = K/V · t = 0.009. (23.3 - 0) = 0.21 | | | | |
| 8 | 26.3 | 1.17 | 0.009 | 0.02 + 0.21 = 0.23 |
| 9 | 29.0 | 1.15 | 0.009 | 0.05 + 0.21 = 0.26 |
| 10 | 32.2 | 1.13 | 0.009 | 0.08 + 0.21 = 0.29 |
| 11 | 35.3 | 1.10 | 0.009 | 0.11 + 0.21 = 0.32 |
| Blood flow change from 300 ml/min to 250 ml/min Second subset closed because of parameter change (4 measurements). Partial Kt/V = K/V · t = 0.008 · (35.3 - 233) = 0.11 Total Kt/V at this treatment time = 0.21 + 0.11 = 0.32 The machine waits for a dead time before starting the measurements again. | | | | |
| 12 | 38.0 | 1.10 | - | - |
| 13 | 40.9 | 1.09 | 0.008 | 0.04 + 0.32 = 0.36 |
| 14 | 44.2 | 1.07 | 0.008 | 0.07 + 0.32 = 0.39 |
| 15 | 47.4 | 1.05 | 0.007 | 0.09 + 0.32 = 0.41 |
| 16 | 50.4 | 1.01 | 0.007 | 0.11 + 0.32 = 0.43 |
| 17 | 53.0 | 1.02 | 0.007 | 0.12 + 0.32 = 0.44 |
| 18 | 56.3 | 1.02 | 0.007 | 0.14 + 0.32 = 0.46 |
| Default closing of third subset (7 measurements). Partial Kt/V = K/V ·t = 0.007 · (56.3 - 35.3) = 0.14 Total Kt/V at this treatment time = 0.32 + 0.14 = 0.46 | | | | |
| ... | ... | ... | ... | ... |
| ... | ... | ... | ... | ... |
| ... | ... | ... | ... | ... |
| ∼ 80 | 240 | ... | ... | ∼ 1.42 |

Because of the position of the measuring unit in the dialy

A kidney substitution treatment machine together with a measuring unit, able to measure the concentration and/or absorbance of any waste product in dialysate, coupled with the flow system of said kidney substitution treatment machine and integrated on the hardware of said kidney substitution treatment machine, or alternatively integrated on a standalone device connected through a external data interface with said kidney substitution treatment machine and coupled with the out flowing dialysate of said kidney substitution treatment machine; that implement an algorithm able to recognize changes on the parameters, which may affect the clearance during a kidney substitution treatment, by analysis of the slope of the timely exponential decay of the concentration of waste products, or by splitting the measured data according to the changes of said parameters; are able to measure the Kt/V value or other adequacy parameters with high accuracy and reproducibility.

During bypass periods the patient is not treated, therefore the data measured during bypass can not be used by the Kt/V measuring functionality. The interface between measuring unit and machine lets the system know about said bypass periods. The data measured during these periods will not be considered and will not affect the accuracy of the function.

During sequential dialysis the dialysate flow is stopped and the weight of the patient is reduced keeping the constant blood osmolarity, meaning that the patient is dried but not dialyzed, therefore the sensor lectures during a sequential period should not be considered by the Kt/V functionality. The interface between measuring unit and machine lets the system know about said sequential periods. The data measured during these periods will not be considered and will not affect the accuracy of the function.

In one of the preferred embodiments during a bypass or a sequential period the UV sensor stops measuring and the current subset is interrupted but not closed, when said period is over the machine waits for a dead time to both let the system stabilize and let the changes arrive from the dialyzer to the position in the flow system where the sensor is coupled. After the dead time the machine may start a procedure to recognize if the absorbance signal has a constant and slow decrease, the fulfilling of this last condition means that the system is stabilized after the bypass and/or sequential period, and the subset which was interrupted can carry on.

The algorithm may work as shown in the following example:

| Measurement n° | Timestamp (min) | UV absorbance | K/V factor | Kt/V |
|---|---|---|---|---|
| 1 | 5.1 | 1.40 | - | - |
| 2 | 8.2 | 1.35 | 0.012 | 0.10 |
| 3 | 11.2 | 1.31 | 0.011 | 0.12 |
| 4 | 15.2 | 1.27 | 0.01 | 0.17 |
| 5 | 17.0 | 1.24 | 0.010 | 0.17 |
| 6 | 20.3 | 1.21 | 0.010 | 0.20 |
| 7 | 23.3 | 1.18 | 0.009 | 0.21 |
| Default closing of first subset (7 measurements). Partial Kt/V = K/V t = 0.009 · (23.3 - 0) = 0.21 | | | | |
| 8 | 26.3 | 1.17 | - | - |
| 9 | 29.0 | 1.15 | 0.009 | 0.05 + 0.21 = 0.26 |
| 10 | 32.2 | 1.13 | 0.009 | 0.08 + 0.21 = 0.29 |
| 11 | 35.3 | 1.10 | 0.009 | 0.11 + 0.21 = 0.32 |
| Bypass and/or sequential period. Second subset interrupted. When the bypass and/or sequential period is over the machine waits for a dead time. When the dead time is over the machine checks that the absorbance signal decreases in a constant an slow basis. The UV-Sensor resumes the measurements. | | | | |
| 12 | 38.0 | 1.10 | 0.008 | 0. 12 + 0.21 = 0.33 |
| 13 | 40.9 | 1.09 | 0.008 | 0.14 + 0.21 = 0.35 |
| 14 | 44.2 | 1.07 | 0.008 | 0.16 + 0.21= 0.37 |
| Default closing of third subset (7 measurements). Partial Kt/V = K/V · t = 0.008 · (44.2 - 23.3) = 0.16 | | | | |
| Total Kt/V at this treatment time = 0.21 + 0.16 = 0.37 | | | | |
| ... | ... | ... | ... | ... |
| ... | ... | ... | ... | ... |
| ... | ... | ... | ... | ... |
| ~80 | 240 | ... | ... | ~ 1.42 |

A kidney substitution treatment machine together with a measuring unit, able to measure the concentration and/or absorbance of any waste product in dialysate, coupled with the flow system of said kidney substitution treatment machine and integrated on the hardware of said kidney substitution treatment machine, or alternatively integrated on a standalone device connected through a external data interface with said kidney substitution treatment machine and coupled with the out flowing dialysate of said kidney substitution treatment machine; that implement an algorithm able to recognize bypass or sequential periods by analysis of the sensor's output signal, or by management of the different events happening in the machine; are able to measure the Kt/V value or other adequacy parameters with high accuracy and reproducibility.

## Claims

1.  A method for measuring the adequacy parameters that are achieved during a kidney substitution treatment,

    - wherein the kidney substitution treatment is provided by a machine, which has an extracorporeal blood system pumping the patient blood at a set blood flow rate through the blood chamber of a dialyzer, divided by a semi-permeable membrane into the blood chamber and a dialyzing fluid chamber, the dialyzing fluid flows at a preset flow rate through the dialyzing fluid system of the machine and collects the waste products from the patient after flowing through the dialyzing fluid chamber of the dialyzer,
    - wherein a device able to measure continuously a kidney substitution treatment related waste product is mounted in the dialyzing fluid system of the kidney substitution treatment machine,
    - wherein the data provided by the kidney substitution treatment machine is used to measure the adequacy parameters at the end of the kidney substitution treatment,
    - wherein the data provided by the device able to measure continuously any kidney substitution treatment waste product is split in subsets to be used to determine the adequacy parameters achieved during the kidney substitution treatment with an algorithm,
    - wherein a data interface is implemented between the kidney substitution treatment machine and the measuring device in order to register machine events to be considered in the algorithm for the determination of the adequacy parameters, and
    - wherein the measuring algorithm is based on a kind of non linear fitting procedure for each of the data subsets with or without considering any kind of event happening on the dialysis machine.

2.  The method according to claim 1, wherein the kidney substitution treatment can be double needle hemodialysis, single needle hemodialysis, single needle cross over hemodialysis, post-dilution hemodiafiltration, pre-dilution hemodiafiltration, pre-post-dilution hemodiafiltration, post-dilution hemofiltration, pre-dilution hemofiltration, pre-post-dilution hemofiltration or sequential hemodialysis.

3.  The method according to claim 1 or 2, wherein the adequacy parameters are Kt/V, single pool Kt/V or equilibrated Kt/V of any waste product present on the dialyzing fluid of any kidney substitution treatment.

4.  The method according to one of the claims 1 to 3, wherein the adequacy parameters are the reduction ratio of any waste product present on the dialyzing fluid of any kidney substitution treatment, the single pool reduction ratio of any waste product present on the dialyzing fluid of any kidney substitution treatment, or the equilibrated reduction ratio of any waste product present on the dialyzing fluid of any kidney substitution treatment.

5.  The method according to one of the claims 1 to 4, wherein the non linear fitting procedure is applied to a single exponential model.

6.  The method according to one of the claims 1 to 4, wherein the non linear fitting procedure is applied to a complex exponential model.

7.  The method according to one of the claims 1 to 6, wherein the machine events considered in the algorithm are any event that can produce a change on the treatment clearance or in the final adequacy parameter.

8.  The method according to one of the claims 1 to 7, wherein the events are blood flow change, dialysate flow change, therapy time change or sequential periods.

9.  The method according to one of the claims 1 to 8, wherein the machine events are detected by the measuring algorithm by analyzing the output signals of the measuring unit.

10. The method according to one of the claims 1 to 9, wherein the measurement is done continuously.

11. The method according to one of the claims 1 to 10, wherein the measuring device is integrated in the kidney substitution treatment machine.

12. The method according to one of the claims 1 to 11, wherein the measuring device is a stand alone device coupled with the flow system of the kidney substitution treatment machine.

13. The method according to one of the claims 1 to 12, wherein the data subsets are based on periods of constant treatment parameters.

14. The method according to claim 13, wherein the constant parameters are blood flow, dialysate flow and treatment time.

15. The method according to one of the claims 1 to 14, wherein the number of subsets maximizes the accuracy of the measurement.

16. The method according to one of the claims 1 to 15, wherein the measurements during bypass periods and sequential periods are not considered on the calculation of the adequacy parameters.


**Patentansprüche**

1. Ein Verfahren zum Messen von Effizienzparametern, die während eine Nierenersatzbehandlung erreicht werden,

- wobei die Nierenersatzbehandlung von einer Maschine vorgenommen wird, die über ein extrakorporales Blutsystem verfügt, das das Blut des Patienten mit einer eingestellten Blutflussrate durch die blutseitige Kammer eines Dialysators pumpt, der durch eine semipermeable Membran in die blutseitige Kammer und eine dialysatseitige Kammer unterteilt ist, die Dialyseflüssigkeit mit einer voreingestellten Flussrate durch das dialysatseitige System der Maschine fließt und die Abfallprodukte von dem Patienten nach dem Durchfluss durch die dialysatseitige Kammer des Dialysators aufnimmt,
- wobei eine Vorrichtung, die befähigt ist, die mit der Nierenersatzbehandlung verbundenen Abfallprodukte kontinuierlich zu messen, in dem dialysatseitigen System der Maschine zur Nierenersatzbehandlung angebracht ist,
- wobei die Daten, die von der Maschine zur Nierenersatzbehandlung geliefert werden, verwendet werden, um die Effizienzparameter am Ende der Nierenersatzbehandlung zu messen,
- wobei die Daten, die von der zur kontinuierlichen Messung jedweden Abfallprodukts der Nierenersatzbehandlung befähigten Vorrichtung geliefert werden, in Untergruppen unterteilt werden, um mittels eines Algorithmus zur Bestimmung der Effizienzparameter verwendet zu werden, die während der Nierenersatzbehandlung erzielt wurden,
- wobei die Datenschnittstelle zwischen der Maschine zur Nierenersatzbehandlung und der Messvorrichtung eingebaut ist, um Vorkommnisse an der Maschine zu registrieren, die in dem Algorithmus zur Bestimmung der Effizienzparameter Berücksichtigung finden sollen, und
- wobei der Messalgorithmus auf einer Art nicht-linearen Fitting-Verfahrens für jede Untergruppe der Daten beruht, mit oder ohne Berücksichtigung jedweden an der Dialysemaschine auftretenden Vorkommnisses.

2. Das Verfahren gemäß Anspruch 1, wobei die Nierenersatzbehandlung eine Hämodialyse über zwei Nadeln, Hämodialyse über eine Nadel, Cross-Over-Hämodialyse mit einer Nadel, Hämodiafiltration mit Postdilution, Hämodiafiltration mit Prädilution, Hämodiafiltration mit Prä-Post-Dilution, Hämofiltration mit Postdilution, Hämofiltration mit Prädilution, Hämofiltration mit Prä-Post-Dilution oder eine sequentielle Hämodialyse sein kann.

3. Das Verfahren gemäß Anspruch 1 oder 2, wobei die Effizienzparameter Kt/V, single-pool Kt/V oder äquilibrierter Kt/V eines jedweden Abfallprodukts in der Dialyseflüssigkeit einer jedweden Nierenersatzbehandlung sind.

4. Das Verfahren gemäß einem der Ansprüche 1 bis 3, wobei die Effizienzparameter die Reduktionsrate eines jedweden Abfallprodukts in der Dialyseflüssigkeit einer jedweden Nierenersatzbehandlung, die Single-Pool-Reduktionsrate eines jedweden Abfallprodukts in der Dialyseflüssigkeit einer jedweden Nierenersatzbehandlung oder die äquilibrierte Reduktionsrate eines jedweden Abfallprodukts in der Dialyseflüssigkeit einer jedweden Nierenersatzbehandlung sind.

5. Das Verfahren gemäß einem der Ansprüche 1 bis 4, wobei das nicht-lineare Fitting-Verfahren auf ein monoexponentielles Modell angewandt wird.

6. Das Verfahren gemäß einem der Ansprüche 1 bis 4, wobei das nicht-lineare Fitting-Verfahren auf ein komplex-exponentielles Modell angewandt wird.

7. Das Verfahren gemäß einem der Ansprüche 1 bis 6, wobei es sich bei den Vorkommnissen an der Maschine, die in dem Algorithmus berücksichtigt werden, um jedwedes Vorkommnis handeln kann, das eine Veränderung in der Clearance-Rate während der Behandlung oder in dem endgültigen Effizienzparameter auslösen kann.

8. Das Verfahren gemäß einem der Ansprüche 1 bis 7, wobei die Vorkommnisse eine Veränderung im Blutfluss, eine Veränderung im Fluss der Dialyseflüssigkeit, eine Veränderung der Behandlungszeit oder sequentielle Perioden sind.

9. Das Verfahren gemäß einem der Ansprüche 1 bis 8, wobei die Vorkommnisse an der Maschine von dem Messalgorithmus durch eine Analyse der Ausgangssignale der Messeinheit festgestellt werden.

10. Das Verfahren gemäß einem der Ansprüche 1 bis 9, wobei die Messung kontinuierlich vorgenommen wird.

11. Das Verfahren gemäß einem der Ansprüche 1 bis 10, wobei die Messvorrichtung in die Maschine zur Nierenersatzbehandlung integriert ist.

12. Das Verfahren gemäß einem der Ansprüche 1 bis 11, wobei die Messvorrichtung ein eigenständiges Gerät ist, das mit dem Flusssystem der Maschine zur Nierenersatzbehandlung gekoppelt ist.

13. Das Verfahren gemäß einem der Ansprüche 1 bis 12, wobei die Untergruppen der Daten auf Perioden konstanter Behandlungsparameter beruhen.

14. Das Verfahren gemäß Anspruch 13, wobei die konstanten Parameter der Blutfluss, der Fluss der Dialyseflüssigkeit und die Behandlungszeit sind.

15. Das Verfahren gemäß einem der Ansprüche 1 bis 14, wobei die Anzahl der Untergruppen die Messgenauigkeit maximieren.

16. Das Verfahren gemäß einem der Ansprüche 1 bis 15, wobei die Messungen während Bypass-Perioden und sequentiellen Perioden keine Berücksichtigung bei der Berechnung der Effizienzparameter finden.

**Revendications**

1. Un procédé pour mesurer des paramètres d'efficacité atteints pendant un traitement de substitution rénale,

- dans lequel le traitement de substitution rénale est fourni par une machine ayant un système du sang extracorporel qui pompe le sang du patient avec un débit de sang fixe à travers la chambre sanguine d'un dialyseur, séparé par une membrane hémiperméable dans la chambre sanguine et une chambre de liquide de dialyse, le liquide de dialyse coule avec un débit fixe à travers le système de dialysat de la machine et collecte les produits de déchet du patient après de couler à travers la chambre de liquide de dialyse du dialyseur,
- dans lequel le dispositif capable de mesurer continuellement un produit de déchet lié au traitement de substitution rénale est monté dans le système de dialysat de la machine pour le traitement de substitution rénale,
- dans lequel les données fournies par la machine pour le traitement de substitution rénale sont usées pour mesurer les paramètres d'efficacité à la fin du traitement de substitution rénale,
- dans lequel les données fournies par le dispositif capable de mesurer continuellement quelconque produit de déchet du traitement de substitution rénale sont divisées dans des sous-ensembles pour être utilisés pour déterminer les paramètres d'efficacité atteints pendant le traitement de substitution rénale avec un algorithme,
- dans lequel l'interface des données est réalisé entre la machine pour le traitement de substitution rénale et le dispositif de mesure pour registrer des évènements de machine pour être considérés dans l'algorithme pour la détermination des paramètres d'efficacité, et

- dans lequel l'algorithme de mesure repose sur une sorte de procédé d'ajustement non-linéaire pour chaque sous-ensemble des données avec ou sans considération de quelconque sorte d'évènement arrivant dans la machine de dialyse.

2. Le procédé selon la revendication 1, dans lequel le traitement de substitution rénale peut être une hémodialyse double-aiguille, hémodialyse simple-aiguille, hémodialyse «cross-over» simple-aiguille, hémodiafiltration post-dilution, hémodiafiltration prédilution, hémodiafiltration pré-post-dilution, hémofiltration post-dilution, hémofiltration prédilution, hémofiltration pré-post-dilution ou une hémodialyse séquentielle.

3. Le procédé selon la revendication 1 ou 2, dans lequel les paramètres d'efficacité sont Kt/V, spKtN ou eKt/V de quelconque produit de déchet présent dans le liquide de dialyse de quelconque traitement de substitution rénale.

4. Le procédé selon une des revendications 1 à 3, dans lequel les paramètres d'efficacité sont le taux de réduction de quelconque produit de déchet présent dans le liquide de dialyse de quelconque traitement de substitution rénale, le taux de réduction d'un seule groupe de quelconque produit de déchet présent dans le liquide de dialyse de quelconque traitement de substitution rénale ou le taux de réduction équilibré de quelconque produit de déchet présent dans le liquide de dialyse de quelconque traitement de substitution rénale.

5. Le procédé selon une des revendications 1 à 4, dans lequel le procédé d'ajustement non-linéaire est appliqué sur un modèle mono-exponentiel.

6. Le procédé selon une des revendications 1 à 4, dans lequel le procédé d'ajustement non-linéaire est appliqué sur un modèle complexe-exponentiel.

7. Le procédé selon une des revendications 1 à 6, dans lequel les évènements de machine considérés dans l'algorithme sont quelconque évènement qui peut produire un changement dans la clairance du traitement ou dans le paramètre d'efficacité final.

8. Le procédé selon une des revendications 1 à 7, dans lequel les évènements sont un changement du flux sanguin, un changement du flux du liquide de dialyse, un changement de la durée de thérapie ou des périodes séquentielles.

9. Le procédé selon une des revendications 1 à 8, dans lequel les évènements de machine sont détectés par un algorithme de mesure en analysant les signales de sortie de l'unité de mesure.

10. Le procédé selon une des revendications 1 à 9, dans lequel la mesure est conduite continuellement.

11. Le procédé selon une des revendications 1 à 10, dans lequel le dispositif de mesure est intégré dans la machine pour le traitement de substitution rénale.

12. Le procédé selon une des revendications 1 à 11, dans lequel le dispositif de mesure est un dispositif autonome couplé avec le système d'écoulement de la machine pour le traitement de substitution rénale.

13. Le procédé selon une des revendications 1 à 12, dans lequel les sous-ensembles de données reposent sur des périodes de paramètres de traitement constants.

14. Le procédé selon la revendication 13, dans lequel les paramètres constants sont le flux sanguin, le flux du liquide de dialyse et la durée de thérapie.

15. Le procédé selon une des revendications 1 à 14, dans lequel le nombre des sous-ensembles maximise la précision de mesure.

16. Le procédé selon une des revendications 1 à 15, dans lequel les mesures pendant les périodes de by-pass et les périodes séquentielles ne sont pas considérées dans le calcul des paramètres d'efficacité.

figure 1

figure 2

*figure 3*

*figure 4*

*figure 5*

*figure 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1083948 B1

- WO 9855166 A

### Non-patent literature cited in the description

- Hemodialysis machines and monitoris. **Polaschegg HD ; Levin NW.** Replacement of renal function by dialysis. Kluwer academic publishers, 2004, 414-418
- **I. Fridolin ; M. Magnusson ; L.-G. Lindberg.** On-line monitoring of solutes in dialysate using absorption of ultraviolet radiation: Technique description. *The International Journal of Artificial Organs,* 2002, vol. 25 (8), 748-761

- **Uhlin F.** Haemodialysis treatment monitored online by ultra violet absorbance. Linköping University Medical Dissertations n° 962. *Department of Medicine and Care Division of Nursing Science & Department of Biomedical Engineering,* 2006